# FASCICULE DE BREVET EUROPEEN

(11) **EP 1 857 098 B1**
(45) Date de publication et mention de la délivrance du brevet: **13.04.2011**
(21) Numéro de dépôt: 07300794.0
(22) Date de dépôt: 14.02.2007
(51) Int. Cl.: A61K 8/88, A61K 8/37, A61Q 1/04

(54) **Composition cosmétique comprenant un ester de polyol(s) et de dimère d'acide gras, un polyamide et de la silice pyrogénée**
Kosmetische Zusammensetzung, die einen Ester von einem Polyol bzw. Polyolen und einen dimeren zweibasigen Fettsäureester, ein Polyamid und pyrogene Kieselsäure umfasst
Cosmetic composition comprising a polyhydric alcohol and fatty acid diacid dimer ester, a polyamide and pyrogenated silica

(30) Priorité: 15.02.2006 FR 0650545
(43) Date de publication de la demande: 21.11.2007
(73) Titulaire: L'Oréal, 75008 Paris (FR)
(72) Inventeur: Jacques, Véronique, 92340, BOURG LA REINE (FR); Schwartz, Véronique, 75014, PARIS (FR)
(74) Mandataire: Le Coupanec, Pascale A.M.P.

(56) Documents cités:
- US-A1- 2003 223 943
- US-A1- 2005 287 103
- DATABASE WPI Week 200377 Derwent Publications Ltd., London, GB; AN 2003-820130 XP002407784 & JP 2003 226609 A (NIPPON SEIKA KK) 12 août 2003 (2003-08-12)
- DATABASE WPI Week 200467 Derwent Publications Ltd., London, GB; AN 2004-680318 XP002407785 & JP 2004 256515 A (NIPPON SEIKA KK) 16 septembre 2004 (2004-09-16)
- HIBI HIROHISA: "OILY COSMETICS CONTAINING 12-HYDROXYSTEARIC ACID AND (HYDROGENATED) DIMER ACID ESTERS" CHEMABS, 18 avril 2003 (2003-04-18), XP002315682

## Description

La présente invention concerne des compositions cosmétiques, notamment destinées au maquillage et/ou au soin des matières kératiniques, en particulier de la peau et/ou des lèvres, associant au moins un ester de polyol(s) et de dimère diacide d'acide gras, ou d'un de ses esters, à au moins un polyamide, et des particules de silice pyrogénée.

Une composition selon l'invention peut, en particulier, être un produit de maquillage et/ou de soin destiné à être appliqué sur les matières kératiniques telles que la peau du corps, par exemple le visage et/ou les lèvres, ou les cils, et être notamment un gloss, un baume à lèvres, un fond de teint, notamment coulé en coupelle, un produit de coloration de la peau, un produit de maquillage des yeux comme un mascara ou un fard à joues ou à paupières, un produit anti-cerne ou un brillant à lèvres.

La présente invention a également pour objet un procédé de maquillage du visage et/ou du corps humain mettant en oeuvre une composition selon l'invention.

De nombreuses compositions cosmétiques existent pour lesquelles des propriétés de brillance et/ou d'effet coloré du film déposé sur la peau et/ou sur les lèvres sont recherchées. Ces propriétés participent généralement à l'effet esthétique désiré.

En particulier, des compositions comprenant une phase grasse gélifiée ou structurée peuvent être utilisées dans des compositions de maquillage des lèvres de type « gloss », qui selon la nature des huiles de phase grasse, peuvent donner un dépôt brillant sur les lèvres.

Toutefois, ces compositions peuvent présenter une dureté insuffisante et/ou être sous une forme liquide ou faiblement visqueuse. Une telle formulation nécessite une attention particulière de l'utilisatrice dans le stockage du récipient contenant la composition afin d'éviter de le renverser. Par ailleurs, ces compositions nécessitent également la mise en oeuvre de moyens d'applications adaptés.

Ainsi, ces formulations sont peu conviviales d'utilisation, et la présence d'accessoires supplémentaires peut augmenter leur coût de production.

Par ailleurs, de telles compositions peuvent également présenter une instabilité au cours du temps se traduisant par une séparation de phase ou synérèse.

Afin d'éviter ces inconvénients, ces compositions peuvent être structurées au moyen d'agents les rendant beaucoup plus dures mais se traduisant alors souvent par une altération de leur brillance initiale.

En conséquence, il existe un besoin de disposer de compositions cosmétiques qui possèdent une consistance suffisante par exemple pour ne pas s'écouler sous son propre poids et par exemple compatibles avec une application avec le doigt, tout en manifestant une fluidité propice à un étalement rapide et facile sur le support considéré, par exemple les lèvres.

Il existe également un besoin de disposer de compositions cosmétiques qui, en outre, n'induisent pas de sensations collantes après ou lors de leur application.

Il existe également un besoin de disposer de compositions cosmétiques qui soient stables au cours du temps, et qui ne soient pas altérées par des variations de température, notamment par exemple liées aux changements d'environnement extérieur/intérieur.

Il existe également un besoin de disposer de compositions qui présentent un aspect translucide et/ou transparent, et qui présentent une brillance améliorée lors de leur application.

La présente invention a pour objet de satisfaire à l'ensemble de ces besoins.

Ainsi, selon un de ses aspects, la présente invention a pour objet une composition cosmétique de maquillage et/ou de soin des matières kératiniques comprenant :
- au moins un ester de polyol(s) et de dimère diacide d'acide gras, ou d'un de ses esters, à
- au moins un polyamide de masse moléculaire moyenne inférieure à 100 000, comportant a) un squelette polymérique ayant des motifs de répétition hydrocarbonés pourvus d'au moins une fonction amide, b) optionnellement une chaîne grasse pendante et/ou au moins une chaîne grasse terminale, et étant liée à ces motifs hydrocarbonés, ledit polyamide étant de formule générale (I) suivante : 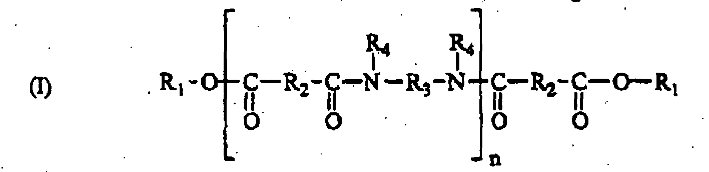 dans laquelle :
   - n désigne un nombre entier de motifs amide tel que le nombre de groupes ester représente de 10 % à 50 % du nombre total des groupes ester et amide ;
   - chacun des symboles R₁ désigne indépendamment un groupe alkyle ou alcényle ayant au moins 4 atomes de carbone, et en particulier de 4 à 24 atomes de carbone ;
   - chacun des symboles R₂ représente indépendamment un groupe hydrocarboné en C₄ à C₄₂ à condition que 50 % des groupes R₂ représentent un groupe hydrocarboné en C₃₀ à C₄₂ ;
   - chacun des symboles R₃ représente indépendamment un groupe organique pourvu d'au moins 2 atomes de carbone, d'atomes d'hydrogène et optionnellement d'un ou plusieurs atomes d'oxygène ou d'azote;
   - et chacun des symboles R₄ représente indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure héterocyclique définie par R₄-N-R₃, avec au moins 50 % des R₄ représentant un atome d'hydrogène, et en outre, des particules de silice pyrogénée.

Selon un autre mode de réalisation, une composition selon l'invention peut posséder, avantageusement, une dureté inférieure ou égale à environ 30 g, et en particulier inférieure ou égale à environ 20 g, mesurée à environ 20 °C.

De manière inattendue, les inventeurs ont observé qu'il était possible de donner satisfaction à l'ensemble des exigences précitées sous réserve d'associer au sein d'une telle composition au moins un polyamide, un ester de polyol(s) et de dimère diacide d'acide gras, ou un de ses esters, et des particules de silice pyrogénée.

Selon un aspect, une composition conforme à l'invention peut, avantageusement, présenter la propriété de ne pas s'écouler sous son propre poids.

Ces compositions présentent également l'avantage d'être transparentes et/ou translucides et peuvent conférer une brillance améliorée au final, notamment sur les lèvres.

En particulier du fait de cette translucidité, l'effet des matières colorantes, en particulier des nacres présentes dans les compositions est exalté dans le conditionnement.

Une composition conforme à l'invention peut être destinée à être appliquée sur les matières kératiniques et notamment la peau du corps et/ou du visage, les lèvres, ou les phanères, tels que les cils.

En particulier, la composition selon l'invention est une composition pour les lèvres.

Elle peut être présentée sous la forme d'un gloss.

Elle peut également se présenter sous une forme compacte.

Ainsi, elle peut être appliquée au doigt ou à l'aide d'un applicateur adapté, tel que par exemple un embout mousse.

Selon un autre de ses aspects, la présente invention a pour objet une utilisation d'au moins un polyamide conforme à l'invention à titre d'agent de structuration d'un ester de polyol(s) et de dimère d'acide diacide gras, ou d'un de ses esters, pour la préparation d'une composition cosmétique. Avantageusement cette composition peut présenter une dureté inférieure à environ 30 g, mesurée à environ 20 °C.

Selon un autre aspect, la présente invention a encore pour objet une utilisation d'une composition cosmétique conforme à l'invention pour l'obtention d'un dépôt brillant.

Selon encore un autre aspect, la présente invention a pour objet un procédé de maquillage et/ou de soin des matières kératiniques, et notamment de la peau et/ou des lèvres, comprenant au moins une étape consistant à appliquer sur au moins une partie desdites matières kératiniques une composition conforme à l'invention.

D'autres caractéristiques, aspects, objets et avantages de la présente invention apparaîtront encore plus clairement à la lecture de la description et des exemples qui suivent.

Selon un aspect, la phase grasse d'une composition selon l'invention est gélifiée ou structurée.

Par « phase grasse gélifiée » au sens de la présente invention, on entend désigner une phase grasse sous forme d'un gel, c'est-à-dire un réseau tridimensionnel qui retient dans ses mailles une quantité importante de phase grasse.

Par « phase grasse structurée », au sens de la présente demande, on entend désigner une phase grasse sous forme d'un gel rigide.

Une composition conforme à l'invention présentant une phase gélifiée ou structurée peut avantageusement présenter une dureté telle que la composition ne s'écoule pas sous son propre poids, à température ambiante et en particulier posséder une dureté inférieure ou égale à environ 30 g, mesurée à environ 20 °C.

### MESURE DE LA DURETÉ

La dureté d'une composition selon l'invention peut être déterminée par une mesure de la force en compression, à 20 °C, à l'aide d'un texturomètre, par exemple vendu sous la dénomination TA-XT2i par la société RHEO, équipé d'un cylindre en inox d'un diamètre de 3 mm se déplaçant à une vitesse de mesure de 0,5 mm/s et pénétrant dans la composition cosmétique à une profondeur de pénétration de 2 mm.

Par exemple, le protocole de mesure peut être tel que défini ci-après.

Un échantillon de composition cosmétique est chauffé à une température de 90-100 °C. L'échantillon fondu est ensuite coulé dans un récipient de 4 mm de profondeur puis est refroidi à une température ambiante d'environ 20-25 °C pendant 24 heures.

L'échantillon est ensuite conservé pendant au moins une heure à 20 °C avant d'effectuer la mesure de dureté.

La valeur de dureté est la force de compression maximale mesurée, divisée par la surface du cylindre du texturomètre en contact avec l'échantillon de composition cosmétique.

Une composition cosmétique conforme à l'invention peut ainsi présenter une dureté variant de 6 g à environ 30 g, en particulier d'environ 10 g à environ 25 g, et plus particulièrement étant d'environ 15 g, mesurée à environ 20 °C.

Parallèlement à cette dureté, une composition selon l'invention peut être avantageusement dénuée de la faculté de s'écouler sous son propre poids.

Au sens de la présente invention, on entend désigner par « composition ne s'écoulant pas sous son propre poids », une composition présentant un comportement rhéologique à température ambiante tel que, lorsque après avoir été chauffée à une température de 90-100 °C, puis coulée dans un récipient par exemple de type cylindrique, d'un diamètre compris entre 2 et 3 cm, d'environ 2-3 cm de profondeur, puis refroidie à une température ambiante d'environ 20-25 °C pendant 24 heures, et que ce récipient est incliné de manière telle que l'axe passant par son orifice forme avec l'axe vertical un angle de 90 °C, pendant une durée d'environ 6 heures, la composition, remplissant au moins la moitié du récipient, ne s'écoule pas à l'extérieur du récipient.

La composition selon l'invention peut présenter un comportement rhéologique particulier, défini notamment par une viscosité pour une gamme de cisaillement définie et mesurée comme suit.

Les mesures peuvent être effectuées au moyen d'un rhéomètre à contrainte imposée, RS 75 de la société ThermoRhéo, équipé d'un bain thermostaté et d'un mobile en acier inoxydable à géométrie cône /plan, le cône ayant un diamètre de 35mm, un angle de 2° et un entrefer (distance entre le plan inférieur - appelé plan stator - sur lequel est déposée la composition et le plan supérieur - appelé plan rotor) de 0,3 mm.

Les mesures sont effectuées à 25°C ± 0,5°C.

L'analyse en régime d'écoulement à l'équilibre consiste à soumettre un échantillon de composition, à partir d'un instant donné, à une contrainte de cisaillement τ instantanée (cisaillement), maintenue constante pendant un temps t (temps d'attente choisi de façon à ce que le régime permanent soit atteint, t = 30s).

Simultanément, on suit l'évolution au cours du temps de la déformation de cisaillement correspondante γ et on enregistre le gradient de cisaillement γ̇ lorsque l'équilibre est atteint.

Dans un premier temps, l'échantillon est mis en température à 25°C pendant 2 min (sans aucun cisaillement appliqué).

Puis l'écoulement à l'équilibre est mesuré en mode contrainte imposée.

On applique des contraintes croissantes à l'échantillon en partant d'une contrainte initiale égale à 0.089 Pa pour arriver à une contrainte finale de 3400 Pa, les contraintes n'étant appliquées qu'une seule fois.

On attend l'obtention d'une valeur stable entre chaque contrainte, le temps d'attente entre chaque contrainte étant de 30 s.

La gamme de gradient de cisaillement va de 10⁻⁴ s⁻¹ à 10³ s⁻¹. Dans la gamme considérée, la valeur maximale de 10³ s⁻¹ doit être prise en compte avec une incertitude de mesure de ± 150 s⁻¹.

L'analyse des résultats se fait au travers de la représentation graphique de l'évolution de la viscosité, notée η, en fonction du gradient de cisaillement, noté γ̇.

La composition selon l'invention peut présenter, avantageusement, un profil rhéologique tel que, pour une gamme de gradient de cisaillement allant 10⁻² à 10 s⁻¹, la viscosité est supérieure à 10⁵ mPa s.

On entend désigner par « température ambiante » ci-dessus, la température de l'environnement dans lequel est disposé une composition et qui peut être fixée, pour des conditions expérimentales reproductibles, entre 20 et 25 °C, mais qui peut être amenée à varier, notamment en fonction des conditions saisonnières et de la nature de l'environnement.

### ESTER DE POLYOL(S) ET DE DIMERE DIACIDE D'ACIDE GRAS

Dans l'expression « ester de polyol(s) et dimère diacide d'acide gras ou d'un de ses esters », on entend désigner par « ou un de ses esters », un des dérivés de ces esters et de polyol(s) et de dimère diacide d'acide gras obtenu soit par réaction de fonction(s) alcool du polyol, non engagée(s) dans des liaisons de type ester avec des fonctions acide du dimère diacide, avec une ou plusieurs fonctions carboxylique de molécules d'acides autre que le dimère diacide ou encore par réaction de fonction(s) acide du dimère diacide, non engagée(s) dans des liaisons de type ester avec des fonctions alcool du polyol, avec des fonctions alcool de molécules d'alcools distinctes du polyol.

Avantageusement, les esters de polyol(s) et de dimère diacide d'acide gras, ou d'un de ses esters, convenant à l'invention peuvent présenter une viscosité, mesurée à environ 25 °C, supérieure ou égale à environ 1500 mPa.s.

La viscosité d'un ester de polyol(s) et de dimère diacide d'acides gras, ou d'un de ses esters selon l'invention peut être mesurée selon tout procédé connu de l'homme de l'art, et notamment selon le procédé conventionnel décrit par la suite.

La viscosité peut être mesurée au moyen d'un viscosimètre cône/plateau ou à plaques parallèles de type ARES (TA-INSTRUMENT) et fonctionnant selon un mode en balayage de cinétique sur une gamme de cisaillement d'environ 1-1000 s⁻¹ pour induire une tension d'écoulement d'environ de 1000 Pa.

Le cône/plateau ou les plaques parallèles peuvent être consitutées d'un matériau choisi parmi l'acier inoxydable, les résines acryliques ou le sulfure de polyphénylène (résine PPS).

Le diamètre cône/plateau peut être de 25 mm (angle de cône 0,10 radiants).

La mesure est réalisée à environ 25 °C.

Avant toute mesure, la stabilité de l'échantillon est vérifiée par le test de la période de balayage dynamique qui permet de déterminer si l'échantillon est stable par lui-même.

La viscosité de cisaillement est déterminée en utilisant la valeur de ETA dans la région du plateau selon l'écoulement.

La période de balayage dynamique est déterminée à une fréquence de 1,0 Hz sur une période de 600 secondes.

Les mesures à vitesse de balayage constante sont réalisées avec une vitesse variant de 1,0 à 1000 s⁻¹, en particulier de 1,0 à 100 s⁻¹.

La viscosité d'un ester de polyol(s) et de dimère diacide d'acides gras, ou d'un de ses esters, convenant à la mise en oeuvre de l'invention peut varier d'environ 1 500 mPa.s à environ 150 000 mPa.s, en particulier d'environ 2 000 mPa.s à environ 150 000 mPa.s, notamment d'environ 15 000 mPa.s à environ 100 000 mPa.s, et en particulier d'environ 30 000 mPa.s à environ 80 000 mPa.s.

Selon un mode particulier de réalisation, un polyol convenant à l'invention peut être avantageusement un dimère diol. 1.

Les esters de dimère diol et de dimère diacide d'acide gras, utilisables dans le cadre de la présente invention sont disponibles commercialement ou peuvent être préparés de manière conventionnelle. Ils peuvent être notamment d'origine végétale et peuvent être obtenus par estérification des dimères diacide avec des dimères diol.

Dans une réaction d'estérification avec un dimère diacide, on peut obtenir un di-carboxylate de polyol(s) qui peut présenter un poids moléculaire moyen en poids, déterminé par chromatographie de perméation de gel (GPC), allant de 2000 à 25 000 g/mol, de préférence entre 2000 et 4000 g/mol.

### Dimère diacide d'acide

Les dimères diacide d'acide gras ou encore dimères diacide peuvent être classiquement obtenus par réaction de polymérisation, notamment de dimérisation intermoléculaire, d'au moins un acide gras insaturé.

Ils peuvent posséder au moins deux groupes carboxyliques.

Comme indiqué précédemment, les fonctions carboxylique du dimère d'acide diacide gras non engagées dans la liaison ester avec le ou les résidus polyols peuvent être engagées dans d'autres liaisons ester avec d'autres fonctions alcool de molécules d'alcool distinctes du ou des polyol(s).

Ces molécules ou résidus d'alcools peuvent être des monoalcools ou des polyols.

A titre d'exemple de résidu d'alcool convenant à la mise en oeuvre de l'invention, on peut mentionner les composés hydrocarbonés comprenant une fonction hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

A titre d'exemple de monoalcool convenant à l'invention, on peut citer, de manière non limitative, le butanol, le pentanol, le propanol, l'hexanol, l'heptanol, l'octanol, le décanol, le dodécanol, l'hexadécanol, l'octadécanol, l'eicosadécanol, le phytostérol, l'isostéarol, le stéarol, le cétol, le béhénol, etc.

Les dimères diacide d'acide gras peuvent dériver, en particulier, de la dimérisation d'un acide gras insaturé, par exemple en C₈ à C₃₄, notamment en C₁₂ à C₂₂, en particulier en C₁₆ à C₂₀, et plus particulièrement en C₁₈.

A titre d'exemples de ces acides gras insaturés, on peut notamment citer l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique, et leurs mélanges.

Selon une variante de réalisation, il peut s'agir plus particulièrement du dimère diacide dont peut également dériver le dimère diol à estérifier. Il peut notamment s'agir de sa forme hydrogénée.

La forme hydrogénée du dimère diacide peut être partielle ou totale, et par exemple correspondre à la forme saturée, plus stable à l'oxydation.

Selon une autre variante de réalisation, il peut s'agir du dimère diacide dérivé de la dimérisation de l'acide linoléique.

Selon un mode de réalisation, le dimère diacide peut être un produit commercialisé, constitué d'un acide dicarboxylique ayant environ 36 atomes de carbone. Ce produit peut contenir également un acide trimérique et un acide monomèrique, dans des proportions qui dépendent du degré de pureté du produit.

Classiquement, on trouve dans le commerce des produits dont la teneur en dimère diacide peut être supérieure à 70 % et d'autres dont la teneur en dimère diacide a pu être ajustée à 90 % ou plus.

On peut également trouver dans le commerce des dimères diacides et notamment des diacides dilinoléiques dont la stabilité vis-à-vis de l'oxydation a été améliorée par hydrogénation des doubles liaisons restant après la réaction de dimérisation.

Dans la présente invention, on peut utiliser tout dimère diacide disponible actuellement dans le commerce.

Dans une réaction d'estérification avec un dimère diacide, le degré moyen d'estérification et le poids moléculaire moyen de l'ester obtenu peuvent être ajustés en faisant varier le rapport du dimerdiol au dimère diacide.

### Polyols

Par le terme « polyol », on entend couvrir tout composé hydrocarboné comprenant au moins deux fonctions hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.

Les chaînes hydrocarbonées peuvent, le cas échéant, être interrompues par la présence d'au moins un hétéroatome, et notamment un atome d'oxygène.

Un polyol ou un ester de polyol convenant à la mise en oeuvre de la présente invention peut comprendre, par exemple, de 2 à 12 fonctions hydroxyle, en particulier de 2 à 8 fonctions hydroxyle, et plus particulièrement de 4 à 6 fonctions hydroxyle.

Le cas échéant, les fonctions hydroxyle, autres que celles déjà engagées dans une liaison ester avec le dimère diacide peuvent être également engagées, toutes ou en partie dans d'autres liaisons ester après réaction avec des molécules d'acides autres que le dimère diacide.

Le polyol ou un de ses esters convenant à la mise en oeuvre de la présente invention peut être, notamment, choisi parmi les alcools linéaires, ramifiés, cycliques ou polycycliques, saturés ou insaturés.

Ainsi, le polyol peut être choisi par exemple parmi un diol, un triol, un tétraol, ou un pentaol, ou un de leurs esters.

Le polyol peut être un diol, ou un de ses esters, par exemple choisi parmi un dimère d'alcool gras, un mono- ou poly-glycérol, un mono- ou poly-alkylène en C₂₋₄ glycol, le 1,4 butanediol, et le pentaérythritol.

A titre d'exemple de diol pouvant également convenir à la mise en oeuvre de l'invention, on peut citer, de manière non exhaustive, le butadiol, le pentadiol, le propanediol, l'hexanediol, l'hexylèneglycol, l'heptanediol, l'octanediol, le nonanediol, le décanediol, l'un-décanediol, le dodécanediol, le tridécanediol, le tétradécanediol, le pentadécanediol, l'hexadécanediol, le nonadécanediol, l'octadecènediol, le cyclohexanediol, le diglycérol, l'érythritol, le pentaérythritol, le xylitol, le sorbitol, l'éthylèneglycol, le xylèneglycol et leurs isomères.

Selon une variante, à titre d'exemple de diol convenant à l'invention, on peut mentionner les dimères diol.

Au sens de la présente invention, on entend plus particulièrement désigner sous le terme « dimère diol » des diols saturés dérivés de l'hydrogénation des dimères diacides correspondants, un dimère diacide étant tel que défini ci-dessus, et en particulier un dimère diacide d'au moins un acide gras insaturé.

En ce qui concerne le dimère diol fabriqué industriellement, il contient également généralement d'autres composants, par exemple, un trimère triol, un monoalcool et des composés de type éther, selon le degré de purification de l'acide dimérique et/ou de l'ester d'alcool inférieur de celui-ci, utilisé comme matière première.

Généralement, les produits dont la teneur en dimère diol est supérieure à 70 % peuvent être utilisés dans la présente invention. Toutefois, il est préférable d'utiliser un dimère diol de grande pureté, tel qu'un composé dont la teneur en dimère diol est supérieure à 90 %.

Ainsi, un dimère diol peut être produit par hydrogénation, par exemple catalytique, d'un dimère diacide, lui-même obtenu par dimérisation d'au moins un acide gras insaturé.

Un acide gras convenant peut être tels que ceux cités précédemment, et notamment en C₈ à C₃₄, en particulier en C₁₂ à C₂₂, en particulier en C₁₆ à C₂₀, et plus particulièrement en C₁₈.

Selon un mode de réalisation particulier, le dimère diol peut dériver de l'hydrogénation du diacide dilinoléique.

Un dimère diol peut, par exemple, être le dilinoléol.

Le dimère diol peut être généralement sous une forme saturée.

A titre d'autre exemple de dimère diol susceptible de convenir à la mise en oeuvre de l'invention, on peut notamment citer le diglycérol.

Ce composé est un dimère de glycérol résultant de la condensation de deux molécules de glycérol avec perte d'une molécule d'eau.

On entend par « diglycérol », l'ensemble des isomères susceptibles de résulter d'une telle condensation, comme par exemple les isomères linéaires, les isomères ramifiés et le cas échéant, les isomères cycliques résultant d'une déshydratation intra-moléculaire d'une molécule de diglycérol.

Le diglycérol peut être obtenu par tout procédé connu de l'homme de l'art et notamment ceux décrits dans le brevet EP 0 750 848.

A titre d'exemple de molécules d'acides susceptibles d'interagir avec une ou plusieurs fonctions hydroxyle du polyol, non engagée(s) dans la liaison ester avec le dimère diacide, on peut mentionner, de manière non limitative, les molécules dérivées de l'acide isostéarique, l'acide béhénique, l'acide phytostérique, l'acide stéarique ou l'acide cétylique.

Un ester convenant à la mise en oeuvre de la présente invention peut être obtenu par réaction d'un polyol ou un de ses esters avec un dimère diacide , et notamment un acide dimerdilinoléique, selon un rapport molaire d'environ 1,0:0,2-1,0.

Un ester susceptible de convenir à la mise en oeuvre de la présente invention peut, notamment, être obtenu par réaction d'un acide dimerdilinoléique avec un dilinoléol, et le cas échéant, au moins un monoalcool additionnel, notamment choisi parmi le béhénol, l'isostéarol, le phytostérol, le stéarol, le cétol et leurs mélanges.

Ainsi, un ester mis en oeuvre dans le cadre de la présente invention peut être utilisé sous la forme d'un mélange de différents esters par exemple.

Un ester convenant à l'invention peut par exemple être obtenu par réaction d'un glycérol, d'un acide isostéarique et d'un acide dimerdilinoléique, notamment, selon un rapport molaire de 1,0:0,2-1,0:0,5-0,9.

A titre d'exemple d'ester d'acide dimerdilinoléique et de polyol(s) ou un de ses esters convenant à l'invention, on peut citer les esters décrits dans les demandes JP 2003-226609, JP 2004-256515 et JP 2005-179377.

Un ester de polyol(s) et de dimère diacide d'acide gras, ou d'un de ses esters, convenant à la mise en oeuvre de la présente invention peut présenter un poids moléculaire variant d'environ 2000 à environ 25 000 g/mol, en particulier d'environ 4 000 à environ 20 000 g/mol, en particulier d'environ 5 000 à environ 20 000 g/mol, en particulier d'environ 7 000 à environ 15 000 g/mol, et plus particulièrement d'environ 8000 à environ 10 000 g/mol.

Selon un mode de réalisation, un ester conforme à l'invention peut comprendre un enchaînement alterné de résidu(s) dimère(s) diacide(s) et de résidu(s) apparenté(s) au(x)dit(s) polyol(s), et notamment au(x)dit(s) diol(s), le(s)dit(s) polyol(s) ou diol(s) étant, par exemple, tel(s) que défini(s) précédemment.

Ainsi, dans une telle configuration, chacune des deux extrémités dudit enchaînement peut porter respectivement un motif OR' et OR", avec R' et R" représentant, indépendamment l'un de l'autre, un atome d'hydrogène ou OR' et OR" représentant, indépendamment l'un de l'autre, un résidu d'un monoalcool hydrocarboné en C₂ à C₃₆, notamment en C₈ à C₂₄, en particulier en C₁₂ à C₂₀, et plus particulièrement en C₁₆ à C₁₈.

Selon un mode de réalisation, R' et R" peuvent représenter tous les deux un atome d'hydrogène.

Selon un mode de réalisation, OR' et OR" peuvent représenter tous les deux un résidu de monoalcool hydrocarboné, identique ou différent.

A titre d'exemple de résidus de monoalcool hydrocarboné OR' et OR" pouvant convenir à l'invention, on peut mentionner les résidus d'alcools gras.

Un ester convenant à l'invention peut être choisi parmi les esters de formules générales (I), (II) ou (IV) décrites ci-après, ou un mélange de ceux-ci.

Selon un mode de réalisation, un ester de polyol(s) et de dimère diacide d'acide gras, ou un de ses esters, pouvant convenir à la mise en oeuvre de la présente invention peut être de formule générale (I) suivante :

R₃-OCO-R₁(-COO-R₂-OCO-R₁)ₙ-COO-R₃ (I)

dans laquelle :
- COR₁CO représente un résidu de dimère diacide d'acide gras,
- OR₂O représente un résidu de dimère d'alcool gras,
- OR₃ représente un résidu de monoalcool hydrocarboné, et
- n est un entier variant de 1 à 15, en particulier de 2 à 10 et plus particulièrement de 5 à 7.

Selon une variante de réalisation, COR₁CO peut représenter un résidu dimerdilinoléate.

Selon une variante de réalisation, OR₂O peut représenter un résidu dimerdilinoléyle.

Par ailleurs, OR₃ peut représenter un résidu de monoalcool hydrocarboné choisi, par exemple, parmi les résidus béhényle, isostéaryle, phytostéryle et leurs mélanges.

Selon un autre variante de réalisation, l'ester d'acide dimerdilinoléique et de polyol(s) et de dimère d'acide diacide gras, ou un de ses esters, pouvant convenir à la mise en oeuvre de l'invention peut être, notamment, de formule générale (II) suivante : 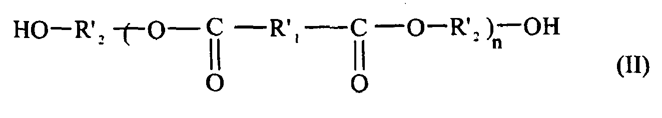 dans laquelle :
- n est un entier variant de 1 à 15, notamment de 2 à 10 et en particulier de 5 à 7,
- COR'₁CO représente un résidu de dimère diacide d'acide gras,
- OR'₂O représente un résidu diglycéryle de formule générale (III) suivante : 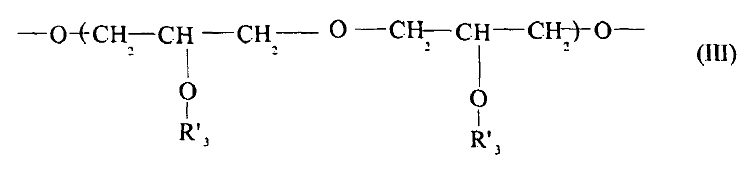 dans laquelle :
   - R'₃ représente H ou OR'₃ représente un résidu d'acide gras.

Selon une variante de réalisation, COR'₁CO peut représenter un résidu dimerdilinoléate.

Selon une variante de réalisation, le résidu d'acide gras figuré par OR'₃ peut être un résidu d'isostéaryle.

Selon un mode de réalisation, un ester d'acide dimerdilinoléique et de polyol(s) et de dimère d'acide diacide gras, ou un de ses esters, pouvant convenir à la mise en oeuvre de la présente invention peut être de formule (IV) suivante :

HO-R₁"-(-OCO-R₂"-COO-R₁"-)ₕ-OH (IV)

dans laquelle :
a) OR₁"O représente un résidu de dimère diol obtenu par hydrogénation d'un acide dimerdilinoléique,
b) COR₂"CO représente un résidu de dimère d'acide diacide gras, et
c) h représente un entier variant de 1 à 9, et en particulier de 2 à 8, et plus particulièrement de 4 à 6.

Selon un mode de réalisation, COR₂"CO peut représenter un résidu dimerdilinoléate

Un ester convenant à l'invention peut notamment être choisi parmi les esters de nomenclature INCI suivante : le copolymère d'isostéarate de polyglyceryle-2 dimerdilinoléate, le bis-béhényl/isostéaryl/phytostéryl dimerdilinoléyle dimerdilinoléate, le dimerdilinoléyle dimerdilinoléate, et leurs mélanges.

De tels composés peuvent être obtenus, par exemple, sous la référence HAILUSCENT ISDA (KOKYU ALCOHOL), PLANDOOL-G, LUSPLAN DD-DA 5 et LUSPLAN DD-DA7, PHY/IS-DA et LUSPLAN DD-DAS (NIPPON FINE CHEMICAL COMPANY, Ltd).

Par exemple, le LUSPLAN DD-DA 5 et LUSPLAN DD-DA7 sont décrits dans la demande FR 03 028 09.

L'ester de polyol(s) et de dimère d'acide diacide gras insaturé ou un de ses esters est généralement ajustée de manière à contrôler la brillance de la composition à une valeur souhaitée.

En l'occurrence, l'ester peut être présent en une teneur variant d'environ 1 à environ 95 %, en poids, en particulier d'environ 5 à environ 85 % en poids, et plus particulièrement d'environ 10 à environ 60 % en poids par rapport au poids total de la composition.

### POLYAMIDE

Une composition selon la présente invention comprend au moins un polymère de masse moléculaire moyenne en poids inférieure à 100 000, notamment inférieure à 50 000, comportant a) un squelette polymérique ayant des motifs de répétition hydrocarbonés pourvus d'au moins une fonction amide, et b) optionnellement au moins une chaîne grasse pendante et/ou au moins une chaîne grasse terminale et étant liées à ces motifs hydrocarbonés «revendication 1».

Par « polymère », on entend au sens de l'invention un composé ayant au moins 2 motifs de répétition, et de préférence au moins 3 motifs de répétition.

Par « motif de répétition hydrocarbonés », on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun d'une à plusieurs fonctions amide avantageusement non pendantes et se trouvant dans le squelette polymérique.

Selon un mode de réalisation, des chaînes grasses pendantes peuvent être liées directement à l'un au moins des atomes d'azote des motifs amide.

Un polyamide convenant à l'invention peut comprendre entre les motifs hydrocarbonés des motifs siliconés ou des motifs oxyalkylénés.

En outre, les chaînes grasses d'un polyamide convenant à la mise en oeuvre de la composition de l'invention peuvent représenter de 40 à 98 % du nombre total des motifs amide et des chaînes grasses, et en particulier de 50 à 95 % du nombre total des motifs amide et des chaînes grasses.

Avantageusement, un polyamide selon l'invention peut présenter une masse moléculaire moyenne en poids inférieure à 100 000, en particulier variant de 1000 à 100 000, en particulier variant de 1000 à 50 000, en particulier variant de 1000 à 30 000, en particulier variant de 2000 à 20 000, et plus particulièrement variant de 2000 à 10 000 g/mol.

Un polyamide convenant à l'invention peut être non soluble dans l'eau, notamment à 25 °C. En particulier, il ne comporte pas de groupe ionique.

Un polyamide convenant à l'invention peut être un polyamide, résultant d'une polycondensation d'un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone).

Un diacide convenant à l'invention peut être par exemple un dimère issu d'acide gras à insaturation éthylénique ayant au moins 16 atomes de carbone, en particulier de 16 à 24 atomes de carbone, comme l'acide oléique, linoléique ou linolénique.

A titre d'exemple de diamine, on peut mentionner l'éthylène diamine, l'hexylène diamine, l'hexaméthylène diamine.

Ces polymères peuvent être par exemple ceux décrits dans le document US-A-5783657 de la société Union Camp.

Le polyamide de l'invention est de formule générale (I) suivante : 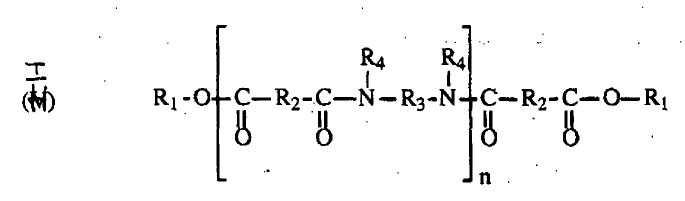 dans laquelle :
- n peut désigner un nombre entier de motifs amide tel que le nombre de groupes ester représente de 10 % à 50 % du nombre total des groupes ester et amide ;
- chacun des symboles R₁ peut désigner indépendamment un groupe alkyle ou alcényle ayant au moins 4 atomes de carbone et en particulier de 4 à 24 atomes de carbone ;
- chacun des symboles R₂ peut représenter indépendamment un groupe hydrocarboné en C₄ à C₄₂, à condition que 50 % des groupes R₂ représentent un groupe hydrocarboné en C₃₀ à C₄₂ ;
- chacun des symboles R₃ peut représenter à chaque occurrence indépendamment un groupe organique pourvu d'au moins 2 atomes de carbone, d'atomes d'hydrogène et optionnellement d'un ou plusieurs atomes d'oxygène ou d'azote ; et
- chacun des symboles R₄ peut représenter indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure hétérocyclique définie par R₄-N-R₃, avec au moins 50 % des R₄ représentant un atome d'hydrogène.

Dans le cas particulier de la formule (I), les chaînes grasses terminales au sens de l'invention sont des chaînes terminales liées au dernier atome d'azote du squelette polyamide.

En particulier, les groupes ester de la formule (I), qui font partie des chaînes grasses terminales et/ou pendantes au sens de l'invention, peuvent représenter de 15 à 40 % du nombre total des groupes ester et amide et en particulier de 20 à 35 %.

De plus, n peut représenter avantageusement un nombre entier allant de 1 à 5 et en particulier supérieur à 2.

R₁ peut être un groupe alkyle en C₁₂ à C₂₂ et en particulier en C₁₆ à C₂₂.

R₂ peut être un groupe hydrocarboné (alkylène) en C₁₀ à C₄₂, et en particulier, 50 % au moins et plus particulièrement au moins 75 % des R₂ peuvent être des groupes hydrocarbonés ayant de 30 à 42 atomes de carbone. Les autres R₂ peuvent être des groupes hydrocarbonés en C₄ à C₁₉ et en particulier en C₈ à C₁₂.

R₃ peut représenter un groupe hydrocarboné en C₂ à C₃₆ ou un groupe polyoxyalkyléné.

En particulier, R₃ peut représenter un groupe hydrocarboné en C₄ à C₁₂. R₄ peut représenter un atome d'hydrogène.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Les polymères de formule (I) peuvent se présenter sous forme de mélanges de polymères. Ces mélanges peuvent en outre contenir un produit de synthèse correspondant à un composé de formule (I) où n vaut 0, c'est-à-dire un diester.

Selon un mode de réalisation, un polyamide convenant à l'invention peut être choisi parmi les copolymères de diacide en C₃₆ condensés sur une éthylène diamine de masse moléculaire en poids d'environ 6 000, et leurs mélanges.

A titre d'exemple de polymères utilisables dans les compositions selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94 °C. Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme polyamide utilisable dans les compositions selon l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid^{®} par les sociétés Général Mills, Inc. et Henkel Corp. (Versamid 930, 744 ou 1655) ou par la société Olin Mathieson Chemical Corp., sous la marque Onamid^{®} notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid^{®} 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Un polyamide convenant à la préparation d'une composition selon l'invention peut présenter une température de ramollissement supérieure à 65°C et pouvant aller jusqu'à 190 °C. En particulier, il peut présenter une température de ramollissement variant de 70 à 130 °C, et en particulier de 80 à 105 °C.

D'une manière générale, les particules de polyamide sont présentes dans une composition selon l'invention en une quantité suffisante pour ajuster la dureté de ladite composition à la valeur requise.

Un polyamide est présent dans une composition selon l'invention en une teneur variant d'environ 0,01 % à environ 4 % en poids par rapport au poids total de la composition.

### SILICE PYROGENEE

La composition selon l'invention peut comprendre, en outre, avantageusement des particules de silice pyrogénée.

Les particules de silice pyrogénée convenant à la mise en oeuvre de l'invention peuvent être hydrophiles ou être traitées en surface afin d'être rendues hydrophobes.

Les silices pyrogénées hydrophiles peuvent être obtenues par hydrolyse à haute température d'un composé volatil du silicium dans une flamme oxhydrique, produisant une silice finement divisée. Les silices hydrophiles peuvent présenter un nombre important de groupements silanol à leur surface.

De telles silices hydrophiles sont par exemple commercialisées sous les dénominations "AEROSIL 130^{®} ", "AEROSIL 200^{®}", "AEROSIL 255^{®}", "AEROSIL 300^{®}", "AEROSIL 380^{®}" par la société Degussa, "CAB-O-SIL HS-5^{®}", "CAB-O-SIL EH-5^{®}", "CAB-O-SIL LM-130^{®}", "CAB-O-SIL MS-55^{®}", "CAB-O-SIL M-5^{®}" par la société Cabot.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812^{®}" par la société Degussa, "CAB-O-SIL TS-530^{®}" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972^{®}", "AEROSIL R974^{®}" par la société Degussa, "CAB-O-SIL TS-610^{®}", "CAB-O-SIL TS-720^{®}" par la société Cabot.

Les particules de silice pyrogénée sont présentes dans une composition conforme à l'invention en une teneur variant de 10 à 30 % en poids par rapport au poids total de la composition.

D'autres composés usuellement utilisés en cosmétique, tels que des phases grasses liquides ou solides, des actifs cosmétiques, des charges ou des matières colorantes, peuvent être additionnellement présents en des quantités ajustées de manière à ce que les propriétés d'une composition conforme à l'invention, notamment en terme de dureté, de brillance et/ou de transparence n'en soient pas substantiellement altérées.

### MILIEU PHYSIOLOGIQUEMENT ACCEPTABLE

Une composition conforme à l'invention comprend un milieu physiologiquement acceptable.

Par « milieu physiologiquement acceptable », on entend désigner un milieu convenant particulièrement à l'application d'une composition selon l'invention sur la peau et/ou les lèvres. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse comprenant, essentiellement d'eau.

Elle peut également comprendre un mélange d'eau et de solvant miscible à l'eau (miscibilité dans l'eau supérieure à 50 % en poids à 25 °C) comme un, ou un mélange de monoalcool(s) inférieur(s) ayant de 1 à 5 atomes de carbone tel(s) que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leurs mélanges.

Selon un autre mode de réalisation, la composition conforme à l'invention peut être anhydre.

Par composition anhydre, on entend une composition comprenant moins de 10 % en poids, en particulier moins de 5 % en poids, en particulier moins de 3 % en poids, en particulier moins de 2 % en poids, et plus particulièrement moins de 1 % en poids d'eau par rapport au poids total de la composition.

### PHASE GRASSE

Une composition cosmétique conforme à la présente invention peut comprendre, outre l'ester de polyol(s) et de dimère diacide d'acide gras, une phase grasse additionnelle, notamment une phase grasse liquide, comprenant une ou plusieurs huiles, et/ou une phase grasse solide à température d'environ 20 - 25 °C et pression atmosphérique, et leur mélange.

On entend par huile, tout corps gras sous forme liquide à température d'environ 20 - 25 °C et à pression atmosphérique. La phase grasse liquide peut, également, contenir outre des huiles, d'autres composés solubilisés dans les huiles tels que des agents gélifiants et/ou structurants.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.

La phase grasse liquide pouvant convenir à la préparation d'une composition cosmétique selon l'invention peut être choisie parmi des huiles volatiles ou non, siliconées ou non, et leurs mélanges.

Au sens de la présente invention, on entend par « huile siliconée », une huile comprenant au moins un atome de silicium, et notamment au moins un groupe Si-O.

On entend par « huile hydrocarbonée », une huile contenant principalement des atomes d'hydrogène et de carbone et éventuellement des atomes d'oxygène, d'azote, de soufre et/ou de phosphore.

### Huiles volatiles

Une composition conforme à l'invention peut comprendre au moins une huile volatile.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les huiles de silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluorométhylcyclopentane, et leurs mélanges.

Il est également possible d'utiliser un mélange des huiles précédemment citées.

Une composition cosmétique selon l'invention peut comprendre au moins une huile volatile en une teneur variant d'environ 1 % à environ 50 % en poids, en particulier variant d'environ 2 % à environ 40 % en poids, et plus particulièrement variant d'environ 5% à environ 30% en poids d'huile volatile par rapport au poids total de la composition.

### Huiles non volatiles

Au sens de la présente invention, on entend par « huile non-volatile », une huile ayant une pression de vapeur inférieure à 0,13 Pa et notamment des huiles de masse molaire élevée. Les huiles non volatiles peuvent être des huiles hydrocarbonées notamment d'origine animale ou d'origine végétale, des huiles d'origine synthétique ou minérale, des huiles siliconées, des huiles fluorées, ou leurs mélanges.

Une composition cosmétique selon la présente invention peut comprendre en outre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile convenant à la mise en oeuvre de l'invention, on peut notamment citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810^{®}, 812^{®} et 818^{®} par la société DYNAMIT NOBEL,
- les huiles hydrocarbonées d'origine minérale ou synthétique comme par exemple :
   - les éthers de synthèse ayant de 10 à 40 atomes de carbone ;
   - les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
   - les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple :
❖ l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
• les esters de polyols, et les esters de pentaétrythritol, comme le tétrahydroxystéarate/tétraisostéarate de dipentaérythritol,
• les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
• les acides gras supérieurs tels que l'acide oléique, l'acide linoléique, l'acide linolénique et leurs mélanges, et
• les carbonates de di-alkyle, les 2 chaînes alkyles pouvant être identiques ou différentes, tel que le dicaprylyl carbonate commercialisé sous la dénomination Cetiol CC®, par Cognis,
• les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,

- et leurs mélanges.

Les huiles non volatiles peuvent être présentes dans une composition selon l'invention en une teneur allant de 5 à 90 % en poids, notamment de 25 % à 80 % en poids, et en particulier de 40 % à 70 % en poids, par rapport au poids total de la composition

### Autres corps gras

Une composition selon l'invention peut également comprendre au moins un corps gras choisi parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope.

Elle peut être hydrocarbonée, fluorée et/ou siliconée et être d'origine animale, végétale, minérale ou synthétique.

Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que « l'EMW-0003 », commercialisé par la société NIPPON SEIROU, les oligomères d'α-oléfine, tel que les polymères « PERFORMA V^{®} 825 », « 103 » et « 260 », commercialisés par la société NEW PHASE TECHNOLOGIES ; les copolymères éthylène-propylène, tel que le « PERFORMALENE^{®} EP 700 », et les cires microcristallines dont le point de fusion est supérieur à 85 °C, tel que les « HI-MIC^{®} 1070 », « 1080 », « 1090 » et « 3080 », commercialisées par NIPPON SEIROU, et leurs mélanges.

Selon un mode de réalisation, la teneur de la ou des cire(s) utilisée(s) dans une composition cosmétique conforme à l'invention peut être inférieure ou égale à environ 5 % en poids par rapport au poids total de la composition.

Selon un autre mode de réalisation, une composition conforme à l'invention peut être exempte de cire.

Une composition cosmétique conforme à la présente invention peut également comprendre au moins un composé pâteux.

Par « pâteux » au sens de la présente invention, on entend un composé gras à changement d'état solide/liquide réversible et comportant à la température de 23 °C une fraction liquide et une fraction solide. On entend également par pâteux, le polylaurate de vinyle.

Un composé pâteux au sens de l'invention peut présenter avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société DOW CORNING sous les noms commerciaux de DC2503^{®} et DC25514^{®} et leurs mélanges.

### MATIERE COLORANTE

Une composition cosmétique conforme à l'invention peut, en outre, comprendre au moins une matière colorante.

Selon un mode de réalisation, une ou des matières colorantes peuvent être ajoutées à une composition cosmétique selon l'invention de manière à ce que la transparence et/ou la translucidité de cette dernière n'en soit pas affectée.

Une telle matière colorante peut être par exemple choisie parmi les matières colorantes hydrosolubles ou non, liposolubles ou non, organiques ou inorganiques, notamment de type pigments ou nacres, classiquement utilisée dans les compositions cosmétiques, les matériaux à effet optique, et leurs mélanges.

Les matières colorantes peuvent être présentes à raison de 0,01 à 40 % en poids, notamment de 0,5 à 25 % en poids par rapport au poids total de la composition cosmétique.

Par pigments, il faut comprendre des particules blanches ou colorées, inorganiques (minérales) ou organiques, insolubles dans une solution aqueuse, destinées à colorer le film résultant.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence « COVERLEAF NS » ou « JS » par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence « PC BALL PC-LL-100 P », ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicétopyrrolopyrroles (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d' oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres « TIMICA », « FLAMENCO » et « DUOCHROME » (sur base de mica) commercialisées par la société ENGELHARD, les nacres « TIMIRON » commercialisées par la société MERCK, les nacres sur base de mica « PRESTIGE » commercialisées par la société ECKART et les nacres sur base de mica synthétique « SUNSHINE » commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone); les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté par exemple commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques.

Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations « STARBRITE 1200 EAC^{®}» par la société SIBERLINE et « METALURE^{®} » par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations « ROTOSAFE 700 » de la société ECKART, les particules d'aluminium enrobé de silice commercialisées sous la dénomination « VISIONAIRE BRIGHT SILVER » de la société ECKART et les particules d'alliage métallique comme des poudres de bronze (alliage cuivre et zinc) enrobé de silice commercialisées sous la dénomination de « VISIONAIRE BRIGHT NATURAL GOLD » de la société ECKART.

Il peut encore s'agir de particules comportant un substrat de verre comme celles commercialisées par la société NIPPON SHEET GLASS sous les dénominations « MICROGLASS METASHINE ».

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination « CHROMAFLAIR » par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination « SICOPEARL » par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂; SnO/TiO₂/SiO₂/TiO₂/SnO ; Fe₂O₃/SiO₂/Fe₂O₃ ; SnO/mica/TiO₂/SiO₂/TiO₂/mica/SnO, des pigments ayant ces structures étant commercialisés sous la dénomination « XIRONA » par la société MERCK (Darmstadt). A titre d'exemple, ces pigments peuvent être les pigments de structure silice/oxyde de titane/oxyde d'étain commercialisés sous le nom « XIRONA MAGIC » par la société MERCK, les pigments de structure silice/oxyde de fer brun commercialisés sous le nom « XIRONA INDIAN SUMMER » par la société MERCK et les pigments de structure silice/oxyde de titane/mica/oxyde d'étain commercialisés sous le nom « XIRONA CARRIBEAN BLUE » par la société MERCK. On peut encore citer les pigments « INFINITE COLORS » de la société SHISEIDO. Selon l'épaisseur et la nature des différentes couches, on obtient différents effets. Ainsi, avec la structure Fe₂O₃/SiO₂/Al/SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination « COLOR GLITTER ».

Comme particules goniochromatiques à cristaux liquides, on peut utiliser par exemple celles vendues par la société CHENIX ainsi que celle commercialisées sous la dénomination « HELICONE^{®} HC » par la société WACKER.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave, le bleu de méthylène.

Selon un mode de réalisation, une composition selon l'invention peut comprendre au moins une matière colorante par exemple choisie parmi les matières colorantes organiques et les matières colorantes inorganiques, telles que les pigments et les nacres, les matériaux à effet optique spécifique et leurs mélanges.

Selon un autre mode de réalisation, la composition selon l'invention est exempte de matière colorante.

### CHARGES

Les compositions cosmétiques conformes à l'invention peuvent également comprendre au moins une charge, de nature organique ou minérale.

Au sens de l'invention, les charges sont distinctes des matières colorantes.

Une ou des charges peuvent être avantageusement ajoutées à une composition cosmétique selon l'invention de telle sorte que ces propriétés, notamment de viscosité, de transparence, de translucidité, n'en soient pas substantiellement affectées.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage. Elles sont distinctes des particules de silice pyrogénée.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et en particulier elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation), les microbilles de résine de silicone (Tospearl ® de Toshiba, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI, et leurs mélanges.

Une charge peut être présente dans une composition cosmétique conforme à l'invention à raison d'environ 0,1 à environ 30 % en poids de charge par rapport au poids total de la composition, de préférence d'environ 0,5 à environ 15 %.

Une charge convenant à l'invention peut être par exemple une charge dont la granulométrie moyenne est inférieure à 100 µm, notamment comprise entre 1 et 50 µm, par exemple entre 4 et 20 µm.

### ADDITIFS

Une composition cosmétique selon l'invention peut également comprendre en outre tout additif usuellement utilisé dans le domaine concerné, choisi parmi des agents filmogènes, et le cas échéant des auxiliaires de filmification, des gommes, des polymères semi-cristallins, des gélifiants, des agents émulsifiants et le cas échéant des agents co-émulsifiants, des agent antioxydants, des huiles essentielles, des conservateurs, des parfums, des actifs cosmétiques, des neutralisants, des agents hydratants, des agents antiseptiques, des vitamines telles que les vitamines B3 ou E et leurs dérivés, des agents protecteurs contre les UV, et leurs mélanges.

Comme actifs cosmétiques utilisables dans les compositions de l'invention, on peut citer les hydratants (polyol comme glycérine), les vitamines (C, A, E, F, B, ou PP), les acides gras essentiels, les huiles essentielles, les céramides, les sphingolipides, les filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

Il relève des opérations de routine de l'homme de l'art d'ajuster la nature et la quantité des additifs présents dans les compositions conformes à l'invention de telle sorte que les propriétés cosmétiques et les propriétés de viscosité désirées de ces dernières n'en soient pas affectées.

Selon un mode de réalisation, une composition conforme à l'invention peut comprendre à titre d'ester de polyol(s) et de dimére diacide d'acide gras, un copolymère dimère dilinoléyle diol/ dimère dilinoléique (ou nom INCI: dimère dilinoléyle dimerdilinoléate), et à titre de mélange de polyamide, un condensat diacide en C₃₆ hydrogène/éthylène diamine, estérifié par de l'alcool stéarylique (poids moléculaire : environ 4000) stabilisé (Uniclear 100 VG^{®}).

Selon une variante de réalisation, une composition selon l'invention peut, en outre, comprendre des particules de silice pyrogénée hydrophobes, traitées en surface par du di-méthylsilane (Aerosil R972^{®}).

La présente invention a également pour objet une utilisation d'au moins un polyamide de masse moléculaire moyenne inférieure à 100 000, comportant a) un squelette polymérique ayant des motifs de répétition hydrocarbonée pourvus d'au moins une fonction amide, b) optionnellement une chaîne grasse pendante et/ou au moins une chaîne grasse terminale, éventuellement fonctionnalisée, ayant de 6 à 120 atomes de carbones, et étant liée à ces motifs hydrocarbonés, à titre d'agent de structuration d'au moins une phase grasse comprenant au moins un ester de polyol(s) et de dimère diacide d'acide gras, ou un de ses esters, pour la préparation d'une composition cosmétique.

Avantageusement, une telle composition peut présenter une dureté inférieure ou égale à 30 g, mesurée à environ 20 °C.

Selon un mode de réalisation, l'ester de polyol(s)et de dimère d'acide diacide gras mis en oeuvre dans l'utilisation conforme à l'invention peuvent être tels que définis précédemment.

Selon un mode de réalisation, le polyamide mis en oeuvre dans l'utilisation conforme à l'invention peut être tel que défini précédemment.

Selon un mode de réalisation, une composition cosmétique selon l'invention peut être préparée selon un procédé comprenant au moins des étapes consistants à :
a) chauffer un mélange d'au moins un ester de polyol(s) et de dimère diacide d'acide gras, ou un de ses esters, et d'au moins un polyamide à une température de fusion dudit polyamide, notamment en présence d'autres composés de type huile et/ou cire, et
b) ajouter, le cas échéant, les autres ingrédients de type matériaux pulvérulents et excipients tels que par exemple les particules de silice pyrogénées en maintenant le mélange obtenu à l'étape a) sous agitation pour obtenir une composition homogène.

La composition peut se présenter sous forme coulée, et par exemple, sous la forme d'un stick ou bâton, sous forme de pâte souple dans une bouillotte, ou sous la forme de coupelle.

Par exemple, elle peut constituer un fond de teint coulé, un fard à joues ou à paupières coulé, notamment coloré, un rouge à lèvres, un brillant pour les lèvres et notamment un gloss, un produit anti-cerne.

Une composition selon l'invention peut notamment se présenter sous la forme d'une composition de maquillage et/ou de soin des lèvres, en particulier un rouge à lèvres, un baume à lèvres, ou un gloss.

Comme indiqué précédemment, une composition cosmétique conforme à l'invention présente avantageusement des propriétés de transparence et de translucidité.

Au sens de l'invention cette propriété de transparence et de translucidité signifie qu'une couche de la composition d'une épaisseur donnée laisse passer une partie de la lumière visible.

Si cette partie de la lumière visible est diffusée, la composition sera définie comme étant une composition translucide, et si au contraire elle n'est pas diffusée, alors la composition sera définie comme étant une composition transparente.

La présente invention est illustrée à l'aide des exemples ci-après. Ces exemples ne constituent en aucun cas une limitation de la présente invention.

### Exemple 1

On prépare un produit de maquillage de type « gloss » pour les lèvres dont la composition est la suivante :

| Ingrédient | Quantité |
|---|---|
| Tri-méllitate de tri-décyle | 34,07 |
| Copolymère dimères dilinolyl diol/dimères dilinoléiques (LUPSLAN DD-DA7 de NIPPON FINE CHEMICAL) | 20 |
| Isoparaffine (6-8 moles d'isobutylène) hydrogénée | 34,07 |
| Condensat diacide en C₃₆ hydrogéné/éthylène diamine, estérifié par alcool stéarylique (poids moléculaire d'environ 4 000) stabilisé (Uniclear 100 VG^{®} de ARIZONA CHEMICAL) | 0,4 |
| Silice pyrogénée hydrophobe, traitée en surface par di-méthylsilane (AEROSIL R972 de Degussa) | 10,5 |
| Parfum | 0,5 |
| Ditertiobutyl 4-hydroxytoluène | 0,06 |
| Mélange de p-hydroxybenzoates d'iso-propyle, isobutyle, N-butyle (40/30/30) | 0,4 |
| Total | 100 |

Le mode opératoire utilisé est le suivant. L'ester de polyol et de dimère diacide d'acide gras (Copolymère dimères dilinolyl diol/dimères dilinoléiques), le polyamide (Uniclear 100), les huiles et les corps gras, les conservateurs, sont mélangés à température de fusion du polyamide, soit environ 105 °C.

Une fois le mélange homogène et le polyamide bien fondu, on ajoute progressivement le parfum, et les particules de silice pyrogénée en maintenant la température à 100-105 °C au Rayneri.

Le mélange est agité jusqu'à homogénéisation complète et obtention d'un mélange transparent.

Pour plus de transparence, on peut soumettre ce mélange au Rayneri sous-vide pour éliminer les éventuelles bulles d'air présentes dans le produit fini.

La composition est ensuite coulée dans un moule.

## Revendications

1. Composition cosmétique, de mouillage et/ou de soin des matières kératiniques, associant
- au moins un ester de polyol(s) et de dimère diacide d'acide gras, ou d' un de ses esters, à
- au moins un polyamide de masse moléculaire moyenne inférieure à 100 000, comportant a) un squelette polymérique ayant des motifs de répétition hydrocarbonée pourvus d'au moins une fonction amide, b) optionnellement une chaîne grasse pendante et/ou au moins une chaîne grasse terminale, et étant liée à ces motifs hydrocarbonés ledit polyamide étant de formule générale (I) suivante : 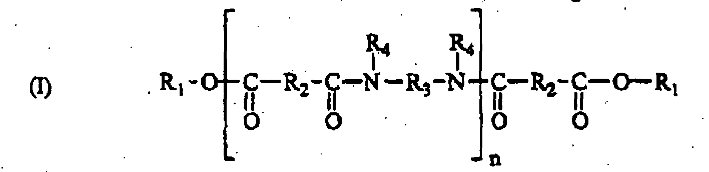 dans laquelle :
- n désigne un nombre entier de motifs amide tel que le nombre de groupes ester représente de 10 % à 50 % du nombre total des groupes ester et amide ;
- chacun des symboles R₁ désigne indépendamment un groupe alkyle ou alcényle ayant au moins 4 atomes de carbone, et en particulier de 4 à 24 atomes de carbone ;
- chacun des symboles R₂ représente indépendamment un groupe hydrocarboné en C₄ à C₄₂ à condition que 50 % des groupes R₂ représentent un groupe hydrocarboné en C₃₀ à C₄₂ ;
- chacun des symboles R₃ représente indépendamment un groupe organique pourvu d'au moins 2 atomes de carbone, d'atomes d'hydrogène et optionnellement d'un ou plusieurs atomes d'oxygène ou d'azote;
- et chacun des symboles R₄ représente indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure héterocyclique définie par R₄-N-R₃, avec au moins 50 % des R₄ représentant un atome d'hydrogène,
ledit polyamide étant présent en une teneur variant de 0,01 à 4 % en poids, par rapport au poids total de la composition,
- et en outre des particules de silice pyrogénée en une teneur variant de 10 à 30 % en poids par rapport au poids total de la composition.

2. Composition selon la revendication 1, ladite composition possédant une dureté inférieure ou égale à 30 g, et en particulier inférieure ou égale à 20 g, mesurée à 20 °C.

3. Composition cosmétique selon la revendication précédente, dans laquelle la dureté varie de 6 g à 30 g, en particulier de 10 g à 25 g, et plus particulièrement étant de 15 g, mesurée à 20 °C.

4. Composition selon l'une quelconque des revendications précédentes, dans laquelle ledit ester possède un poids moléculaire variant de 2000 à 25 000 g/mol, en particulier de 5000 à 20 000 g/mol, en particulier de 7000 à 15 000 g/mol, et plus particulièrement de 8000 g/mol à 10 000 g/mol.

5. Composition selon l'une quelconque des revendications précédentes, dans laquelle l'acide gras est un acide gras insaturé en C₈ à C₃₄, notamment en C₁₂ à C₂₂ en particulier en C₁₆ à C₂₀ et plus particulièrement en C₁₈.

6. Composition selon la revendication précédente, dans laquelle l'acide gras insaturé est choisi parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique, et leurs mélanges.

7. Composition selon l'une quelconque des revendications précédentes, dans laquelle le dimère diacide dérive de la dimérisation de l'acide linoléique.

8. Composition selon l'une quelconque des revendications précédentes, dans laquelle le polyol est un diol.

9. Composition selon la revendication précédente, dans laquelle le diol est choisi parmi un dimère d'alcool gras, un mono- ou poly-glycérol, un mono- ou poly-alkylène en C2-4 glycol, le 1,4 butanediol, et le pentacrythritol

10. Composition selon la revendication 8 ou 9, dans laquelle le diol dérive de l'hydrogénation d'un dimère diacide d'au moins un acide gras insaturé.

11. Composition selon l'une quelconque des revendications précédentes, dans laquelle ledit ester est choisi parmi les esters de formules générales (I), (II) ou (IV) suivantes :
R₃-OCO-R₁(-COO-R₂-OCO-R₁)ₙ-COO-R₃ (I)
dans laquelle :
- COR₁CO représente un résidu dimère diacide d'acide gras,
- OR₂O représente un résidu de dimère d'alcool gras,
- OR₃ représente un résidu de monoalcool hydrocarboné, et
- n est un entier variant de 1 à 15, ou 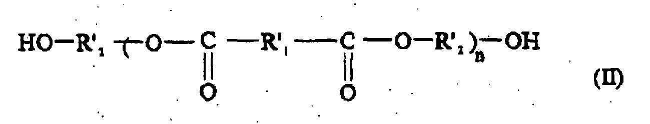
dans laquelle :
- n est un entier variant de 1 à 15,
- COR'₁CO représente un résidu de dimère diacide d'acide gras,
- OR'₂O représente un résidu diglycéryle de formule générale (III) suivante : 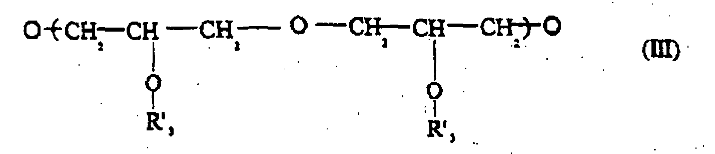 dans laquelle :
- R'₃ représente H ou OR'₃ représente un résidu d' acide gras, ou un mélange de ceux-ci, ou
HO-R₁"-(-OCO-R₂"-COO-R₁"-)_{n-O}H (IV)
dans laquelle :
- OR₁"O représente un résidu de dimère diol obtenu par hydrogénation d'un acide dimerdilinoléique,
- COR₂"CO représente un résidu dimère diacide d'acide gras, et
- h représente un entier variant de 1 à 9.

12. Composition selon la revendication précédente, dans laquelle :
COR₁CO représente un résidu dimerdilinoléate,
- OR₂O représente un résidu dimerdilinoléyle, et
- OR₃ représente un résidu de monoalcool hydrocarboné choisi parmi les résidus béhényle, isostéaryle, phytostéryle et leurs mélanges.

13. Composition selon la revendication 11, dans laquelle :
- COR₁'CO représente un résidu dimerdilinoléate, et
- le résidu d'acide gras figuré par OR'₃ est un résidu d'isastéaryle.

14. Composition cosmétique selon la revendication 11, dans laquelle COR₂"CO représente un résidu dimère dilinoléate.

15. Composition selon l'une quelconque des revendications précédentes, dans laquelle ledit ester est choisi parmi les esters de nomenclature INCI suivante : le copolymère d'isostéarate de polyglycéryle-2/dimerdilinoléate, le bis-béhényl/isostéaryl/phytostéryl dimerdilinoléyle dimerdilinoléate, le dimerdilinoléyle dimerdilinoléate, et leurs mélanges.

16. Composition selon l'une quelconque des revendications précédentes, dans laquelle l'ester de polyol(s) et de dimère diacide d'acides gras, ou un de ses esters, est présent en une teneur variant de 1 à 95 % en poids, en particulier de 5 à 85 % en poids, et plus particulièrement de 10 % à 60 % en poids par rapport au poids total de la composition.

17. Composition selon l'une quelconque des revendications précédentes, dans laquelle les chaînes grasses dudit polyamide représentent de 40 à 98 %, et en particulier de 50 à 95 % du nombre total des motifs amide et des chaînes grasses.

18. Composition selon l'une quelconque des revendications précédentes, dans laquelle la masse moléculaire moyenne du polyamide varie de 1000 à 100 000, en particulier de 1000 à 50 000, en particulier de 1 000 à 30 000, en particulier de 2 000 à 20 000, et plus particulièrement de 2 000 à 10 000 g/mol.

19. Composition selon l'une quelconque des revendication précédentes, dans laquelle R1 est un groupe alkyle en C₁₂ à C₂₂.

20. Composition selon l'une quelconque des revendications précédentes, dans laquelle 50 % des R₂ sont des groupes hydrocarbonés ayant de 30 à 42 atomes de carbone.

21. Composition selon l'une quelconque des revendications précédentes, dans laquelle le polyamide est choisi parmi les copolymères de diacide en C₃₆ condensés, sur une éthylène diamine de masse moléculaire en poids d'environ 6 000, et leurs mélanges.

22. Composition selon l'une quelconque des revendications précédentes, dans laquelle les particules de silice pyrogénée sont choisies parmi des particules de silice pyrogénée hydrophile et des particules de silice pyrogénée hydrophobe.

23. Composition selon l'une quelconque des revendications précédentes, dans laquelle les particules de silice pyrogénée sont présentes en une teneur variant de 10 à 20 % en poids, et plus particulièrement de 10 à 15 % en poids par rapport au poids total de la composition.

24. Composition selon l'une quelconque des revendications précédentes, comprenant en outre au moins une phase grasse additionnelle choisie parmi une phase grasse liquide, une phase grasse solide, et leur mélange.

25. Composition selon l'une quelconque des revendications précédentes, ladite composition comprenant une teneur en cire inférieure ou égale à 5. % en poids par rapport au poids total de la composition.

26. Composition, selon l'une quelconque des revendications précédentes, comprenant en outre au moins une matière colorante.

27. Composition selon l'une quelconque des revendications précédentes, ladite composition étant anhydre.

28. Composition selon l'une quelconque des revendications précédente, ladite composition étant une composition de maquillage et/ou de soin des lèvres.

29. Composition selon l'une quelconque des revendications précédents, ladite composition étant présentée sous la forme d'un gloss.

30. Composition selon l'une quelconque des revendications précédentes, ladite composition étant présentée sous une forme compacte.

31. Utilisation d'au moins un polyamide de masse moléculaire moyenne inférieure à 100 000, comportant a) un squelette polymérique ayant des motifs de répétition hydrocarbonée pourvus d'au moins une fonction amide, b) optionnellement une chaîne grasse pendante et/ou au moins une chaîne grasse terminale, et étant liée à ces motifs hydrocarbonés, ledit polyamide étant de formule générale (I) suivante : 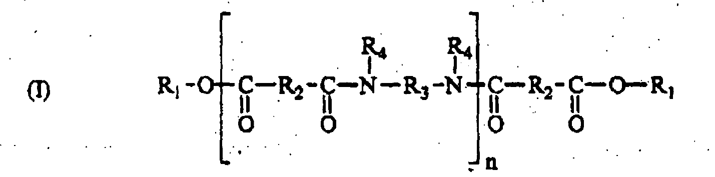 dans laquelle :
- n désigne un nombre entier de motifs amide tel que le nombre de groupes ester représente de 10 % à 50 % du nombre total des groupes ester et amide ;
- chacun des symboles R₁ désigne indépendamment un groupe alkyle ou alcényle ayant au moins 4 atomes de carbone, et en particulier de 4 à 24 atomes de carbone ;
- chacun des symboles R₂ représente indépendamment un groupe hydrocarboné en C₄ à C₄₂ à condition que 50 % des groupes R₂ représentent un groupe hydrocarboné en C₃₀ à C₄₂ ;
- chacun des symboles R₃ représente indépendamment un groupe organique pourvu d'au moins 2 atomes de carbone, d'atomes d'hydrogène et optionnellement d'un ou plusieurs atomes d'oxygène ou d'azote ;
- et chacun des symboles R₄ représente indépendamment un atome d'hydrogène, un groupe alkyl en C₁ à C₁₀ ou une liaison directe à R₃ ou à un autre R₄ de sortie que l'atome d'azote auquel sont liés à la fois R₃ et R₄ fasse partie d'une structure hétérocyclique définie par R₄-N-R₃, avec au moins 50 % des R₄ représentant un atome d'hydrogène, et étant présent en une teneur variant de 0,01 à 4 % en poids par rapport au poids total de la composition,
à titre d'agent de structuration dans une composition comprenant au moins un ester de polyol(s) et de dimère d'acide diacide gras, ou un de ses esters, et des particules de silice pyrogénée en une teneur variant de 10 à 30 % en poids par rapport au poids total de la composition.

32. Utilisation selon la revendication précédente, dans laquelle ledit ester est tel que défini selon l'une quelconque des revendications 4 à 15.

33. Utilisation selon la revendication 31 ou 32, dans laquelle le polyamide est tel que défini selon l'une quelconque des revendications 1 et 17 à 20.

34. Procédé de maquillage et/ou de soin des matières kératiniques comprenant au moins une étape consistant à appliquer sur au moins une partie desdites matières kératiniques composition telle que définie selon l'une quelconque des revendications 1 à 30.

## Claims

1. A cosmetic composition for making up and/or caring for keratin materials, combining
- at least one ester of polyol(s) and of fatty diacid dimer, or an ester thereof, with
- at least one polyamide with an average molecular mass of less than 100,000, comprising a) a polymer backbone with hydrocarbon-based repeating units containing at least one amide function, b) optionally a pendent fatty chain and/or at least one terminal fatty chain, and being linked to these hydrocarbon-based units, said polyamide being of general formular (I) below: 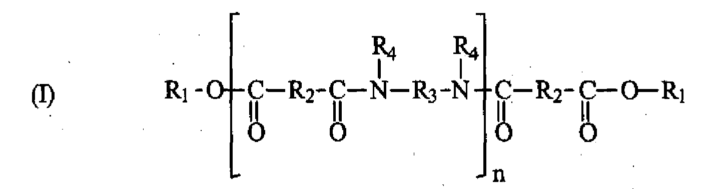 in which:
- n denotes a whole number of amide units such that the number of ester groups represents from 10% to 50% of the total number of ester and amide groups;
- each of the symbols R₁ independently denotes an alkyl or alkenyl group containing at least 4 carbon atoms, and in particular from 4 to 24 carbon atoms;
- each of the symbols R₂ independently represents a C₄ to C₄₂ hydrocarbon-based group, on condition that 50% of the groups R₂ represent a C₃₀ to C₄₂ hydrocarbon-based group;
- each of the symbols R₃ independently represents an organic group containing at least two carbon atoms, hydrogen atoms and optionally one or more oxygen or nitrogen atoms;
- and each of the symbols R₄ independently represents a hydrogen atom, a C₁-C₁₀ alkyl group or a direct bond to R₃ or to another R₄ such that the nitrogen atom to which R₃ and R₄ are both bonded forms part of a heterocyclic structure defined by R₄-N-R₃, with at least 50% of the R₄ representing a hydrogen atom,
said polyamide being present in a content ranging from 0.01% to 4% in weight, relative to the total weight of the composition,
- and further fumed silica particles in a content ranging from 10% to 30% by weight relative to the total weight of the composition.

2. The composition according to claim 1, said composition having a hardness of 30 g, and in particular less than or equal to 20 g, measured at 20°C.

3. The composition according to the preceding claim, in which the hardness ranges from 6 g to 30 g, in particular from 10 g to 25 g, and more particularly being of 15 g, measured at 20° C.

4. The composition according to anyone of the preceding claims, in which said ester has a molecular weight ranging from 2000 to 25,000 g/mol, in particular from 5 000 to 20,000 g/mol, in particular from 7 000 to 15,000 g/mol, and more particularly from 8 000 g/mol to 10,000 g/mol.

5. The composition according to anyone of the preceding claims, in which the fatty acid is a C₈ to C₃₄ unsaturated fatty acid, notably in C₁₂ to C₂₂, in particular in C₁₆ to C₂₀ and more particularly in C₁₈.

6. The composition according to the preceding claim, in which the unsaturated fatty acid is selected from the group consisting of undecenoic acid, linderic acid, myristoleic acid, palmitoleic acid, oleic acid, linoleic acid, elaidinic acid, gadolenoic acid, eicosapentaenoic acid, docosahexaenoic acid, erucic acid, brassidic acid and arachidonic acid, and mixtures thereof.

7. The composition according to anyone of the preceding claims, in which the diacid dimer is derived from the dimerization of linoleic acid.

8. The composition according to anyone of the preceding claims, in which the polyol is a diol.

9. The composition according to the preceding claim, in which the diol is selected from the group consisting of a fatty alcohol dimer, a monoglycerol or polyglycerol, a C₂-C₄ monoalkylene or polyalkylene glycol, 1,4-butanediol and pentaerythritol.

10. The composition according to claim 8 or 9, in which the diol is derived from the hydrogenation of a diacid dimer of at least one unsaturated fatty acid.

11. The composition according to anyone of the preceding claims, in which said ester is chosen from the esters of formula (I), (II) or (IV) below:
R₃-OCO-R₁(-COO-R₂-OCO-R₁)ₙ-COO-R₃ (I)
in which:
- COR₁CO represents a fatty diacid dimer residue,
- OR₂O represents a fatty alcohol dimer residue,
- OR₃ represents a hydrocarbon-based monoalcohol residue, and
- n is an integer ranging from 1 to 15, or 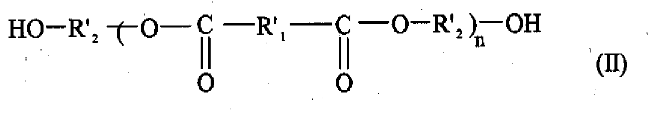 in which:
- n is an integer ranging from 1 to 15,
- COR'₁CO represents a fatty diacid dimer residue,
- OR'₂O represents a diglyceryl residue of general formula (III) below: 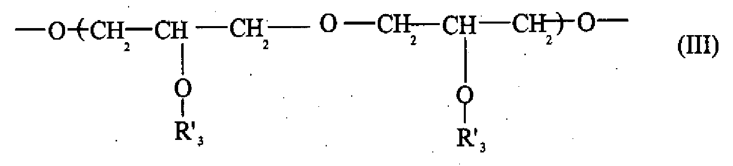 in which:
R'₃ represents H or OR'₃ represents a fatty acid residue, or a mixture thereof, or
HO-R₁"-(-OCO-R₂"-COO-R₁"-)ₕ-OH (IV)
in which:
- OR₁"O represents a diol dimer residue obtained by hydrogenation of a dimerdilinoleic acid,
- COR₂"CO represents a fatty diacid dimer residue, and
- h represents an integer ranging from 1 to 9.

12. The composition according to the preceding claim, in which:
- COR₁CO represents a dimerdilinoleate residue,
- OR₂O represents a dimerdilinoleyl residue, and
- OR₃ represents a hydrocarbon-based monoalcohol residue selected from the group consisting ofbehenyl, isostearyl and phytosteryl residues, and mixtures thereof.

13. The composition according to claim 11, in which:
- COR₁'CO represents a dimerdilinoleate residue, and
- the fatty acid residue featured by OR'₃ is an isostearyl residue.

14. The composition according to claim 11, in which COR₂"CO represents a dilinoleate dimer residue.

15. The composition according to anyone of the preceding claims, in which said ester is selected from the group consisting of the esters having the following INCI nomenclature: polyglyceryl-2 isostearate/dimerdilinoleate copolymer, bis-behenyl/isostearyl/phytosteryl dimerdilinoleyl dimerdilinoleate, dimerdilinoleyl dimerdilinoleate, and mixtures thereof.

16. The composition according to anyone of the preceding claims, in which the ester of polyol(s) and of fatty diacid dimer, or an ester thereof, is present in a content ranging from 1% to 95% by weight in particular from 5 to 85% by weight, and more particularly from 10% to 60% by weight relative to the total weight of the composition.

17. The composition according to anyone of the preceding claims, in which the fatty chains of said polyamide represent from 40% to 98%, and in particular from 50 to 95% of the total number of amide units and of fatty chains.

18. The composition according to anyone of the preceding claims, in which the average molecular mass of the polyamide ranges from 1000 to 100,000, in particular from 1 000 to 50,000, in particular from 1 000 to 30,000, in particular from 2 000 to 20,000 and more particularly from 2 000 to 10,000 g/mol.

19. The composition according to anyone of the preceding claims, in which R₁ is a C₁₂-C₂₂ alkyl group.

20. The composition according to anyone of the preceding claims, in which 50% of the R₂ are hydrocarbon-based groups containing from 30 to 42 carbon atoms.

21. The composition according to anyone of the preceding claims, in which the polyamide is selected from the group consisting of copolymers of C₃₆ diacids condensed onto an ethylenediamine with a weight-average molecular mass of about 6000, and mixtures thereof.

22. The composition according to anyone of the preceding claims, in which the fumed silica particles are selected from the group consisting of hydrophilic fumed silica particles and hydrophobic fumed silica particles.

23. The composition according to anyone of the preceding claims, in which the fumed silica particles are present in a content ranging from 10 to 20% by weight, and more particularly from 10 to 15% by weight relative to the total weight of the composition.

24. The composition according to anyone of the preceding claims, also comprising at least one additional fatty phase selected from the group consisting of a liquid fatty phase and a solid fatty phase, and mixtures thereof

25. The composition according to anyone of the preceding claims, said composition comprising a wax content of less than or equal to 5% by weight relative to the total weight of the composition.

26. The composition according to anyone of the preceding claims, also comprising at least one dyestuff.

27. The composition according to anyone of the preceding claims, said composition being anhydrous.

28. The composition according to anyone of the preceding claims, said composition being a makeup and/or care composition for the lips.

29. The composition according to anyone of the preceding claims, said composition being in the form of a gloss.

30. The composition according to anyone of the preceding claims, said composition being in a compact form.

31. Use of at least one polyamide with an average molecular mass of less than 100,000, comprising a) a polymer backbone with hydrocarbon-based repeating units containing at least one amide function, b) optionally a pendent fatty chain and/or at least one terminal fatty chain, and being linked to these hydrocarbon-based units, said polyamide being of general formula (I) below: 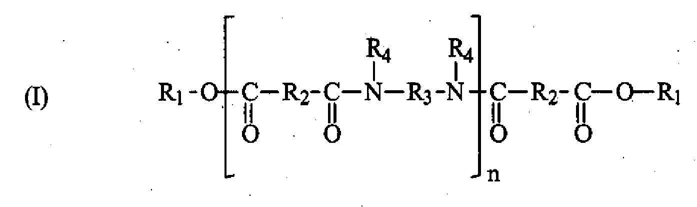 in which:
- n denotes a whole number of amide units such that the number of ester groups represents from 10% to 50% of the total number of ester and amide groups;
- each of the symbols R₁ independently denotes an alkyl or alkenyl group containing at least 4 carbon atoms, and in particular from 4 to 24 carbon atoms;
- each of the symbols R₂ independently represents a C₄ to C₄₂ hydrocarbon-based group, on condition that 50% of the groups R₂ represent a C₃₀ to C₄₂ hydrocarbon-based group;
- each of the symbols R₃ independently represents an organic group containing at least two carbon atoms, hydrogen atoms and optionally one or more oxygen or nitrogen atoms;
- and each of the symbols R₄ independently represents a hydrogen atom, a C₁-C₁₀ alkyl group or a direct bond to R₃ or to another R₄ such that the nitrogen atom to which R₃ and R₄ are both bonded forms part of a heterocyclic structure defined by R₄-N-R₃, with at least 50% of the R₄ representing a hydrogen atom,
said polyamide being present in a content ranging from 0.01 % to 4% in weight, relative to the total weight of the composition,
as a structuring agent in a composition comprising at least one ester of polyol(s) of fatty diacid dimer, or an ester thereof, and fumed silica particles in a content ranging from 10% to 30% by weight relative to the total weight of the composition.

32. The use according to the preceding claim, in which said ester is as defined according to anyone of the claims 4 to 15.

33. The use according to claim 1 or 32, in which the polyamide is as defined according to anyone of the claims 1 and 17 to 20.

34. A process for making up and/or caring for keratin materials, comprising at least one step that consists in applying to at least part of said keratin materials a composition as defined according to anyone of the claims 1 to 30.

## Patentansprüche

1. Kosmetische Schmink- und/oder Pflegezusammensetzung der Keratinsubstanzen, die folgendes kombiniert:
- mindestens einen Ester von Polyol(en) und von dimerer Fett-Disäüre, oder einen ihrer Ester, mit
- mindestens einem Polyamid einer mittleren Molekülmasse von kleiner als 100.000, umfassend: a) ein Polymerskelett mit Kohlenwasserstoff-Wiederholungseinheiten, die mit mindestens einer Amidfunktion versehen sind, b) gegebenenfalls eine Fett-Seitenkette und/oder mindestens eine endständige Fettkette, und die mit diesen Kohlenwasserstoffeinheiten verknüpft ist, wobei das Polyamid die folgende allgemeine Formel (I) aufweist: 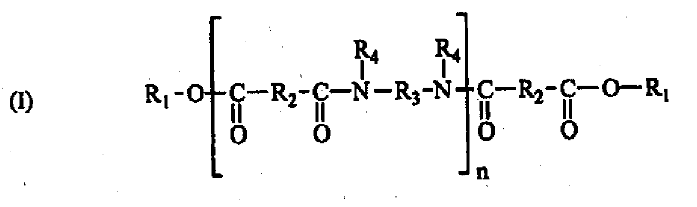 wobei:
- n eine ganze Zahl von Amideinheiten bezeichnet, derart, dass die Zahl von Estergruppen 10 % bis 50 % der Gesamtzahl der Ester- und Amidgruppen darstellt;
- die Symbole R₁ jeweils unabhängig eine Alkylgruppe oder Alkenylgruppe mit mindestens 4 Kohlenstoffatomen, und insbesondere mit 4 bis 24 Kohlenstoffatomen bezeichnen;
- die Symbole R₂ jeweils unabhängig eine C₄- bis C₄₂-Kohlenwasserstoffgruppe darstellen, mit der Maßgabe, dass 50 % der Gruppen R₂ eine C₃₀- bis C₄₂-Kohlenwasserstoffgruppe darstellen;
- die Symbole R₃ jeweils unabhängig eine organische Gruppe darstellen, die mit mindestens 2 Kohlenstoffatomen, Wasserstoffatomen und gegebenenfalls mit einem oder mehreren Sauerstoffatomen oder Stickstoffatomen versehen ist;
- und die Symbole R₄ jeweils unabhängig ein Wasserstoffatom, eine C₁- bis C₁₀-Alkylgruppe oder eine direkte Bindung zu R₃ oder zu einem weiteren R₄ darstellen, derart, dass das Stickstoffatom, an das R₃ und R₄ gleichzeitig gebunden sind, Teil einer heterocyclischen Struktur ist, die durch R₄-N-R₃ definiert ist, wobei mindestens 50 % von R₄ ein Wasserstoffatom darstellen,
wobei das Polyamid in einem Gehalt vorhanden ist, der von 0,01 bis 4 Gew.-% variiert, bezogen auf das Gesamtgewicht der Zusammensetzung,
- und weiterhin pyrogene Kieselsäureteilchen in einem Gehalt, der von 10 bis 30 Gew.-%, bezogen auf das Gesamtgewicht der Zusammensetzung, variiert.

2. Zusammensetzung nach Anspruch 1, wobei die Zusammensetzung eine Härte von kleiner oder gleich 30 g, und insbesondere von kleiner oder gleich 20 g, gemessen bei 20 °C aufweist.

3. Zusammensetzung nach dem vorhergehenden Anspruch, wobei die Härte von 6 g bis 30 g, insbesondere von 10 g bis 25 g variiert und noch spezieller 15 g beträgt, gemessen bei 20 °C.

4. Zusammensetzung nach einem der vorhergehenden Ansprüche, wobei der Ester ein Molekulargewicht besitzt, das von 2000 bis 25.000 g/mol, insbesondere von 5000 bis 20000 g/mol, insbesondere von 7000 bis 15.000 g/mol und noch spezieller von 8000 g/mol bis 10.000 g/mol variiert.

5. Zusammensetzung nach einem der vorhergehenden Ansprüche, wobei die Fettsäure eine ungesättigte C₈- bis C₃₄-Fettsäure, insbesondere eine C₁₂- bis C₂₂-Fettsäure, insbesondere eine C₁₆- bis C₂₀-Fettsäure und noch spezieller eine C₁₈-Fettsäure ist.

6. Zusammensetzung nach dem vorhergehenden Anspruch, wobei die ungesättigte Fettsäure ausgewählt ist aus Undecensäure, Lindersäure, Myristolsäure, Palmitolsäure, Ölsäure, Linolsäure, Elaidinsäure, Gadoleinsäure, Eicosapentaensäure, Docosahexaensäure, Erucasäure, Brassidinsäure, Arachidonsäure und ihren Gemischen.

7. Zusammensetzung nach einem der vorhergehenden Ansprüche, wobei sich die dimere Disäure von der Dimerisation der Linolsäure ableitet.

8. Zusammensetzung nach einem der vorhergehenden Ansprüche, wobei das Polyol ein Diol ist.

9. Zusammensetzung nach dem vorhergehenden Anspruch, wobei das Diol ausgewählt ist aus einem dimeren Fettalkohol, einem Mono- oder Polyglycerol, einem Mono- oder Poly-C₂₋₄-alkylenglycol, 1,4-Butandiol und Pentaerythrit.

10. Zusammensetzung nach Anspruch 8 oder 9, wobei sich das Diol von der Hydrierung von einer dimeren Disäure von mindestens einer ungesättigten Fettsäure ableitet.

11. Zusammensetzung nach einem der vorhergehenden Ansprüche, wobei der Ester aus den Estern der folgenden allgemeinen Formeln (I), (II) oder (IV) ausgewählt ist:
R₃-OCO-R₁(-COO-R₂-OCO-R₁)ₙ-COO-R₃ (I)
wobei
- COR₁CO einen dimeren Fett-Disäurerest darstellt,
- OR₂O einen Rest von dimerem Fettalkohol darstellt,
- OR₃ einen hydrocarbonietten Monoalkoholrest darstellt, und 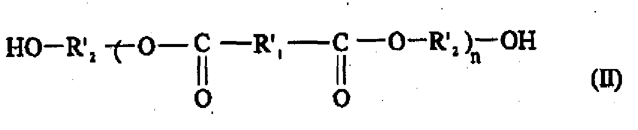
wobei
- n eine ganze Zahl ist, die von 1 bis 15 variiert,
- COR'₁CO einen Rest von dimerer Fett-Disäure darstellt,
- OR'₂O einen Diglycerylrest der folgenden allgemeinen Formel (III) darstellt: 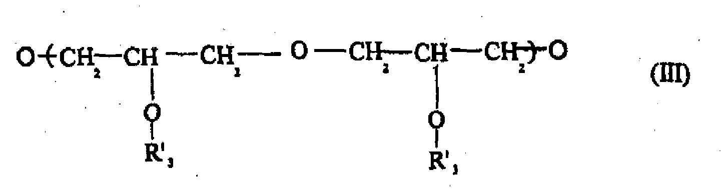
wobei
- R'₃ H darstellt oder OR'₃ eine Fettsäurerest oder ein Gemisch von diesen darstellt; oder
HO-R₁"-(OCO-R₂"-COO-R₁"-)ₕ-OH (IV)
wobei
- OR₁"O einen Rest von dimerem Diol darstellt, der durch Hydrierung einer dimeren Dilinolsäure erhalten wurde,
- COR₂"CO einen dimeren Fett-Disäurerest darstellt, und
- h eine ganze Zahl ist, die von 1 bis 9 variiert.

12. Zusammensetzung nach dem vorhergehenden Anspruch, wobei
- COR₁CO einen dimeren Dilinoleatrest darstellt,
- OR₂O einen dimeren Dilinoleylrest darstellt,
- OR₃ einen Rest von hydrocarboniertem Monoalkohol darstellt, der aus Behenyl-, Isostearyl-, Phytostearylresten und ihren Gemischen ausgewählt ist.

13. Zusammensetzung nach Anspruch 11, wobei
- COR₁'CO einen dimeren Dilinoleatrest darstellt, und
- der durch OR'₃ dargestellte Fettsäurerest ein Isostearylrest ist.

14. Kosmetische Zusammensetzung nach Anspruch 11, wobei COR₂"CO einen dimeren Dilinoleatrest darstellt.

15. Zusammensetzung nach einem der vorhergehenden Ansprüche, wobei der Ester ausgewählt ist aus Estern der folgenden INCI-Nomenklatur: Polyglyceryl-2-isostearat/dimeres Dilinoleat-Copolymer, Bisbehenyl/Isostearyl/Phytostearyl-dimeres Dilinoleyl-dimeres Dilinoleat, dimeres Dilinoleyl-dimeres Dilinoleat und ihren Gemischen.

16. Zusammensetzung nach einem der vorhergehenden Ansprüche, wobei der Ester von Polyol(en) und von dimerer Fett-Disäure, oder einer ihrer Ester in einem Gehalt vorhanden ist, der von 1 bis 95 Gew.-% variiert, insbesondere von 5 bis 85 Gew.-% und noch spezieller von 10 bis 60 Gew.-% variiert, bezogen auf das Gesamtgewicht der Zusammensetzung.

17. Zusammensetzung nach einem der vorhergehenden Ansprüche, wobei die Fettketten des Polyamids 40 bis 98 % und insbesondere 50 bis 95 % der Gesamtzahl der Amideinheiten und Fettketten darstellen.

18. Zusammensetzung nach einem der vorhergehenden Ansprüche, wobei die mittlere Molekülmasse des Polyamids von 1000 bis 100.000, insbesondere von 1000 bis 50.000, insbesondere von 1000 bis 30.000, insbesondere von 2000 bis 20.000 und noch spezieller von 2000 bis 10.000 g/mol variiert.

19. Zusammensetzung nach einem der vorhergehenden Ansprüche, wobei R1 eine C₁₂- bis C₂₂-Alkylgruppe ist.

20. Zusammensetzung nach einem der vorhergehenden Ansprüche, wobei 50 % von R₂ Kohlenwasserstoffgruppen mit 30 bis 42 Kohlenstoffatomen sind.

21. Zusammensetzung nach einem der vorhergehenden Ansprüche, wobei das Polyamid ausgewählt ist aus C₃₆-Disäure-Copolymeren, die über ein Ethylendiamin mit einer Gewichtsmolekülmasse von etwa 6000 kondensiert sind, und ihren Gemischen.

22. Zusammensetzung nach einem der vorhergehenden Ansprüche, wobei die Teilchen von pyrogener Kieselsäure aus Teilchen von hydrophiler pyrogener Kieselsäure und aus Teilchen von hydrophober pyrogener Kieselsäure ausgewählt sind.

23. Zusammensetzung nach einem der vorhergehenden Ansprüche, wobei die Teilchen von pyrogener Kieselsäure in einem Gehalt vorhanden sind, der von 10 bis 20 Gew.-% und spezieller von 10 bis 15 Gew.-%, bezogen auf das Gesamtgewicht der Zusammensetzung, variiert.

24. Zusammensetzung nach einem der vorhergehenden Ansprüche, die weiterhin mindestens eine zusätzliche Fettphase umfasst, die ausgewählt ist aus einer flüssigen Fettphase, einer festen Fettphase und ihren Gemischen.

25. Zusammensetzung nach einem der vorhergehenden Ansprüche, wobei die Zusammensetzung einen Gehalt an Wachs von kleiner oder gleich 5 Gew.-% bezogen auf das Gesamtgewicht der Zusammensetzung umfasst.

26. Zusammensetzung nach einem der vorhergehenden Ansprüche, die weiterhin mindestens einen Farbstoff umfasst.

27. Zusammensetzung nach einem der vorhergehenden Ansprüche, wobei die Zusammensetzung wasserfrei ist.

28. Zusammensetzung nach einem der vorhergehenden Ansprüche, wobei die Zusammensetzung eine Schmink- und/oder Pflegezusammensetzung für die Lippen ist.

29. Zusammensetzung nach einem der vorhergehenden Ansprüche, wobei die Zusammensetzung in der Form eines Gloss dargereicht wird.

30. Zusammensetzung nach einem der vorhergehenden Ansprüche, wobei die Zusammensetzung in einer kompakten Form dargereicht wird.

31. Verwendung von mindestens einem Polyamid mit einer mittleren Molekülmasse von kleiner als 100.000, umfassend a) ein Polymerskelett mit Kohlenwasserstoff-Wiederholungseinheiten, die mit mindestens einer Amidfunktion versehen sind, b) gegebenenfalls eine Fett-Seitenkette und/oder eine endständige Fettkette, und die mit diesen Kohlenwasserstoffeinheiten verknüpft ist, wobei das Polyamid die allgemeine Formel (I) aufweist: 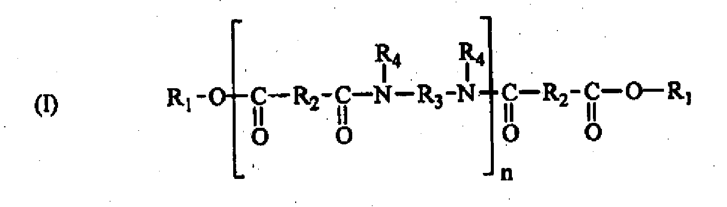 wobei:
- n eine ganze Zahl von Amideinheiten bezeichnet, derart, dass die Zahl von Estergruppen 10 % bis 50 % der Gesamtzahl der Ester- und Amidgruppen darstellt;
- die Symbole R₁ jeweils unabhängig eine Alkylgruppe oder Alkenylgruppe mit mindestens 4 Kohlenstoffatomen bezeichnet, und insbesondere mit 4 bis 24 Kohlenstoffatomen;
- die Symbole R₂ jeweils unabhängig eine C₄- bis C₄₂-Kohlenwasserstoffgruppe darstellt, mit der Maßgabe, dass 50 % der Gruppen R₂ eine C₃₀- bis C₄₂-Kohlenwasserstoffgruppe darstellen;
- die Symbole R₃ jeweils unabhängig eine organische Gruppe darstellt, die mit mindestens 2 Kohlenstoffatomen, Wasserstoffatomen und gegebenenfalls mit einem oder mehreren Sauerstoffatomen oder Stickstoffatomen versehen ist;
- und die Symbole R₄ jeweils unabhängig ein Wasserstoffatom, eine C₁- bis C₁₀-Alkylgruppe oder eine direkte Bindung zu R₃ oder zu einem weiteren R₄ darstellen, derart, dass das Stickstoffatom, an das R₃ und R₄ gleichzeitig gebunden sind, Teil einer heterocyclischen Struktur ist, die durch R₄-N-R₃ definiert ist, wobei mindestens 50 % von R₄ ein Wasserstoffatom darstellen und in einem Gehalt vorhanden sind, der von 0,01 bis 4 Gew.-% variiert, bezogen auf das Gesamtgewicht der Zusammensetzung,
als strukturierendes Mittel in einer Zusammensetzung, die mindestens einen Ester von Polyol(en) und von dimerer Fett-Disäure, oder einen ihrer Ester, und pyrogene Kieselsäureteilchen in einem Gehalt, der von 10 bis 30 Gew.-%, bezogen auf das Gesamtgewicht der Zusammensetzung variiert, umfasst.

32. Verwendung nach dem vorhergehenden Anspruch, wobei der Ester so ist, wie nach einem der Ansprüche 4 bis 15 definiert.

33. Verwendung nach Anspruch 31 oder 32, wobei das Polyamid so ist, wie nach einem der Ansprüche 1 und 17 bis 20 definiert.

34. Verfahren zum Schminken und/oder Pflegen der Keratinsubstanzen, umfassend mindestens einen Schritt bestehend aus dem Auftragen auf mindestens einen Teil der Keratinsubstanzen einer Zusammensetzung wie nach einem der Ansprüche 1 bis 30 definiert.
